# EUROPÄISCHE PATENTANMELDUNG

(11) **EP 4 763 260 A1**
(43) Veröffentlichungstag der Anmeldung: **24.06.2026**
(21) Anmeldenummer: 25222102.3
(22) Anmeldetag: 10.12.2025
(51) Int. Cl.: A61M 25/00, A61M 25/10

(54) **BEREITSTELLUNGSANORDNUNG FÜR DIE ANGIOPLASTIE UND VERFAHREN ZUR HERSTELLUNG EINER BEREITSTELLUNGSANORDNUNG**

(30) Priorität: 17.12.2024 DE 102024138259
(71) Anmelder: B. Braun Melsungen AG, 34212 Melsungen (DE)
(72) Erfinder: ENGELIEN, Eberhard, 21521 Wohltorf (DE); VAN HUYNH, Qui, 14052 Berlin (DE); MOOSIG, Volker, 13595 Berlin (DE); REICH, Henrike, 12439 Berlin (DE)
(74) Vertreter: Winter, Brandl - Partnerschaft mbB

(57) **Zusammenfassung**

Offenbart ist eine Bereitstellungsanordnung (1; 101; 201; 301; 401) für eine perkutane transluminale Angioplastie, mit einem zu sterilisierenden oder sterilisierten Angioplastie-Katheter (2), der einseitig an einem flächigen Träger (4; 104; 204; 304; 404), in einer oder in mehreren Windungen (6, 8, 10), gehalten ist, und der an einem vorbestimmt proximalen Endabschnitt einen Hub (12) und an einem vorbestimmt distalen Endabschnitt wenigstens einen Ballon (14) aufweist, wobei der Träger (4; 104; 204; 304; 404) eine Vertiefung (16; 116; 216; 316; 416) hat, in welcher der Angioplastie-Katheter (2) aufgenommen ist. Offenbart ist darüber hinaus ein Verfahren zur Herstellung einer Bereitstellungsanordnung.

## Beschreibung

Die Offenbarung betrifft eine Bereitstellungsanordnung für die perkutane transluminale Angioplastie mit einem Träger und einem darin gehaltenen, zu sterilisierenden oder sterilisierten Angioplastie-Katheter, welcher endseitig einerseits einen Hub und andererseits wenigstens einen Ballon aufweist. Die Offenbarung betrifft zudem ein Verfahren zur Herstellung einer Bereitstellungsanordnung für die perkutane transluminale Angioplastie.

Angioplastie-Katheter werden in Sterilverpackungen verpackt, darin sterilisiert und anschließend bereitgestellt. Die Sterilverpackung ermöglicht dabei zum einen eine Sterilisation und gewährleistet zum anderen die Sterilität des Katheters bei geeigneter Lagerung bis zu seiner Bereitstellung und Anwendung. Es sollte generell eine Rekontamination des sterilisierten Angioplastie-Katheters nach der Sterilisation bis zu seiner Anwendung ausgeschlossen sein.

In aus dem Stand der Technik bekannter Weise - wie beispielsweise bei dem Produkt SeQuent^{®} NEO der Anmelderin - werden Angioplastie-Katheter in einem spiralförmig gewickelten Kunststoffschlauch, einem sogenannten "Dispenser", gehalten und in einem Sterilbeutel bereitgestellt.

In der Herstellung erfolgt die Verpackung dieses Angioplastie-Katheters, indem er mit seiner den Katheter-Ballon tragenden Spitze in den sich linear erstreckenden Dispenser, der etwas länger als der Angioplastie-Katheter ist, eingefädelt wird und eingeschoben wird, bis der Anschluss oder Hub des Angioplastie-Katheters in Anlage mit der Einführöffnung des Dispensers gerät. Der eingeschobene Angioplastie-Katheter ist dann in dem Dispenser aufgenommen und gegen Beschädigung und Knick geschützt. In Folge wird der Dispenser in zwei bis vier spiralförmige Windungen gelegt/ gewickelt und die Windungen werden mittels Clips fixiert. Der so gewickelte Dispenser wird in Folge in einen Beutel, den sogenannten "Peelbeutel", eingelegt und der Beutel wird verschlossen, insbesondere versiegelt bzw. verschweißt. Der beschriebene Herstellungsprozess ist nur mit relativ hohem Aufwand automatisierbar.

In einem alternativen Herstellungsprozess wird zunächst der Dispenser in spiralförmige Windungen gelegt/gewickelt und fixiert und anschließend der Angioplastie-Katheter in den so gewundenen Dispenser eingeschoben.

Auf dem Peelbeutel sind alle wichtigen Informationen zum enthaltenen Angioplastie-Katheter abgebildet. Das sind z.B. der Katheter-Typ, seine Artikelnummer, Charge, sein Herstellungsdatum und sein Verfalldatum. Als weitere relevante Daten sind zudem der Durchmesser des Führungsdrahtes, der Nenndurchmesser und die Länge des Ballons, sowie der Nenndruck inklusive dem maximal zulässigen Druck (rated burst pressure), des Ballons aufgedruckt. Zudem ist eine Tabelle dargestellt, die das Druck-Durchmesser-Verhalten des Ballons, das sogenannte "Compliance Chart", zeigt, wobei der Nenndruck und der maximal zulässige Druck besonders hervorgehoben sind.

Bei der Durchführung der perkutanen transluminalen Angioplastie wird dem Operateur der Angioplastie-Katheter bereitgestellt, indem der Peelbeutel von einer Assistenz geöffnet wird. Der Operateur entnimmt dem sterilen Inneren des Peelbeutels den durch die Clips spiralförmig gehaltenen Dispenser und zieht den etwa 1,5 bis 2m langen Angioplastie-Katheter aus dem Dispenser heraus. Zuletzt wird dabei die Spitze des Angioplastie-Katheters aus dem Dispenser gezogen, die der Operateur in Folge über die Schleuse des arteriellen Zugangs in den Patienten einführt.

Am Dispenser-System nachteilig ist die große Menge Kunststoff, die für die Verpackung und Bereitstellung des Angioplastie-Katheters benötigt wird. Die Verpackung, das heißt der Peelbeutel und der Dispenser, macht etwa 95 % des Kunststoffes am Produkt aus. Eine Wiederverwendung ist nicht möglich.

Nachteilig ist auch, dass der Operateur den Angioplastie-Katheter immer erst vollständig herausziehen muss, bevor er an die Katheter-Spitze gelangt, um diese beispielsweise zu spülen und/oder den Katheter auf den Führungsdraht auffädeln zu können. Das bringt mit sich, dass der Angioplastie-Katheter bereits in seiner vollen Länge ungeschützt vorliegt, selbst wenn die Aufgabe erst einmal nur darin besteht, die Katheter-Spitze auf den Führungsdraht aufzufädeln und anschließend in die Schleuse am Zugang des Patienten einzuführen. Der Operateur selbst oder eine Assistenz muss somit darauf achten, dass der meterlange Rest des herausgezogenen Angioplastie-Katheters sicher verortet/ gehalten/ gegen Beschädigung oder Verschmutzung geschützt ist. Hinzu kommt, dass am Hub zudem noch eine Spritze angeschlossen sein kann, mit welcher der Katheter evakuiert wird, d.h. mit welcher in dem Katheter ein Vakuum (oder quasi-Vakuum) gezogen wird. Dies erschwert die sichere Handhabung des Katheters weiter. Neben der eigentlichen Kernaufgabe, die Angioplastie durchzuführen, stellt das einen Aufwand und eine Fehlerquelle dar.

Hinzu kommt, dass während der Angioplastie eine Vielzahl von Kathetern zum Einsatz kommt oder kommen kann. Zu nennen sind beispielsweise der Führungskatheter zur Gabe von Kontrastmittel, mehrere Ballonkatheter bei einer sequentiellen, schrittweisen Aufweitung einer Stenose oder bei Mehrfach-Stenosen an einer Furkation, sowie der jeweilige Angioplastie-Katheter zum Setzen des Stents. Wird jeder dieser Katheter per Dispenser bereitgestellt, fällt eine erhebliche Menge an Kunststoff an, die im OP gehandhabt werden muss. Insbesondere müssen die auf den Peelbeuteln abgebildeten Nennwerte und Compliance Charts, bzw. die Peelbeutel selbst, gut verwahrt sein, sodass die Informationen für den Operateur stets verlässlich und übersichtlich zugänglich sind. Ergänzend sollte die Information auch an dem Dispenser vorgesehen sein, da ansonsten die Identifizierung des Angioplastie-Katheters und die Bestimmung seiner Spezifikationen zumindest erschwert ist, sobald der Dispenser mit dem darin enthaltenen Katheter aus dem Peelbeutel entnommen wurden.

Es ist demgegenüber die Aufgabe der Offenbarung, die Nachteile aus dem Stand der Technik zu vermeiden oder wenigstens zu mildern. Insbesondere besteht eine Aufgabe darin, eine Bereitstellungsanordnung für die Angioplastie zu schaffen, die dem Anwender eine variantenreichere Entnahme eines Angioplastie-Katheters ermöglicht. Eine weitere Aufgabe ist, ein Verfahren zur Herstellung einer derartigen Bereitstellungsanordnung zu schaffen, welches es erlaubt, den Aufwand zur Automatisierung zu verringern, sowie insbesondere den Einsatz von Ressourcen zu verringern sowie nachhaltigere Materialien zu verwenden.

Die erste Aufgabe wird durch eine Bereitstellungsanordnung mit den Merkmalen des Anspruchs 1 und die zweite Aufgabe wird durch ein Verfahren mit den Merkmalen des Anspruchs 14 gelöst. Vorteilhafte Weiterbildungen sind Gegenstand der Unteransprüche.

Ein Grundgedanke der Offenbarung sieht vor, einen zu sterilisierenden oder sterilisierten Angioplastie-Katheter an einer Vertiefung eines flächigen Trägers bereitzustellen. Um Platz bei der Bereitstellung zu sparen, ist der Angioplastie-Katheter in der Vertiefung gewunden, das heißt in einer oder in mehreren Windungen aufgenommen. Die Vertiefung ist einseitig am flächigen Träger ausgebildet, das heißt, sie ist von einer Seite offen zugänglich. So kann ein Operateur oder Anwender den Träger ablegen und von der genannten Seite aus den Angioplastie-Katheter an unterschiedlichen Punkten oder Bereichen isoliert oder gemeinsam aufgreifen, insbesondere an einem Hub oder an den Windungen oder an einer Spitze des Katheters. Der Operateur gelangt somit bei der Vorbereitung und bei der Durchführung der Angioplastie gezielt an jeden relevanten Abschnitt des Angioplastie-Katheters, insbesondere an die wichtigen Endabschnitte Hub und Katheter-Spitze, ohne dass er dafür den Angioplastie-Katheter vollständig vom Träger entnehmen/ herausziehen muss, wie das beim Stand der Technik mit Dispenser-Technologie der Fall ist.

Eine offenbarungsgemäße Bereitstellungsanordnung für eine perkutane transluminale Angioplastie hat einen flächigen Träger und einen zu sterilisierenden oder sterilisierten Angioplastie-Katheter. Der Angioplastie-Katheter ist an einer Seite des flächigen Trägers in einer oder in mehreren Windungen gehalten. Der Angioplastie-Katheter hat offenbarungsgemäß einen proximalen Endabschnitt mit einem Hub oder Anschluss und einen distalen Endabschnitt, insbesondere eine Katheter-Spitze, mit wenigstens einem Katheter-Ballon. Offenbarungsgemäß hat der flächige Träger eine Vertiefung, in der der Angioplastie-Katheter aufgenommen ist, sodass der Operateur den Träger sicher ablegen kann und den Angioplastie-Katheter an unterschiedlichen Punkten isoliert oder gemeinsam aufgreifen kann, ohne dafür den Angioplastie-Katheter vollständig vom Träger entnehmen/ herausziehen zu müssen. So ist eine Bereitstellungsanordnung für die perkutane transluminale Angioplastie geschaffen, die dem Anwender eine variantenreiche Entnahme des Angioplastie-Katheters ermöglicht.

Gemäß einer bevorzugten Weiterbildung ist der Träger der Bereitstellungsanordnung einstückig ausgestaltet.

Der Begriff der Einstückigkeit des Trägers bedeutet im Rahmen der Offenbarung insbesondere, dass der Träger nicht aus Teilen oder Stücken zusammengesetzt oder zusammensetzbar ist, die miteinander formschlüssig oder kraftschlüssig oder stoffschlüssig verbunden oder verbindbar sind. In anderen Worten ist unter der Einstückigkeit des Trägers insbesondere eine einmaterialige, integrale, monolithische Fertigung und Ausbildung des Trägers zu verstehen.

Gemäß einer bevorzugten Weiterbildung ist die Vertiefung des Trägers derart ausgestaltet oder geformt, dass der Angioplastie-Katheter allein durch die Ausgestaltung oder Formgebung der Vertiefung in der Vertiefung gehalten ist. In anderen Worten sind keine zusätzlichen Teile oder Stücke - wie beispielsweise eine zusätzliche Abdeckung oder Deckplatte oder ein zusätzliches Deckelement, die/ das die Vertiefung begrenzt oder abdeckt - vorgesehen, um den Angioplastie-Katheter in der Vertiefung zu halten.

Gemäß einer Weiterbildung sind am Träger wenigstens drei Stützpunkte vorgesehen, an denen der Katheter aufgenommen, gehalten und/ oder fixiert ist. Die Stützpunkte können als Vorsprünge eines Randes oder Randabschnittes der Vertiefung, als Halter, als Lasche, als Tasche oder als Clip vorgesehen sein.

Vorzugsweise ist die Vertiefung oder sind die Stützpunkte mit Bezug zum Angioplastie-Katheter nach innen, das heißt hin zum Angioplastie-Katheter, zumindest abschnittsweise gewölbt oder mit zum Zentrum hin gerichteten Vorsprüngen versehen, um so ein Herausrutschen des Angioplastie-Katheters aus der Vertiefung oder aus den Stützpunkten zu vermeiden.

Gemäß einer Weiterbildung ist der Angioplastie-Katheter am Träger derart aufgenommen und gehalten, dass seine Entnahme durch Greifen am Hub und Herausziehen des Hubs möglich ist, und/ oder dass seine Entnahme in einem/ als Ganzes möglich ist und/ oder dass eine gesonderte Entnahme des Hubs und/ oder des Ballons möglich ist.

Jede der Entnahmevarianten bietet dem Operateur ein kontrolliertes Handling des Angioplastie-Katheters, insbesondere des Hubs oder proximalen Endabschnitts und/ oder des Ballons oder distalen Endabschnitts.

Im Falle der Entnahme in einem/ als Ganzes kann der Nutzer oder Operateur den Angioplastie-Katheter aufgewickelt entnehmen, dann beispielsweise eine Spritze zum Evakuieren anschließen, den distalen Endabschnitt/ die Spitze/ den Ballon auf einen Führungsdraht auffädeln und anschließend den Angioplastie-Katheter abwickeln und gestreckt ablegen. Während dieser Handhabung des aufgewickelten Angioplastie-Katheters hat der Nutzer eine bessere Kontrolle über den gesamten Angioplastie-Katheter, als wenn er den Angioplastie-Katheter zuerst streckt und ablegt und anschließend die Spritze anschließt und den distalen Endabschnitt/ die Spitze/ den Ballon auf den Führungsdraht auffädelt.

Gemäß einer bevorzugten Weiterbildung weist die eine oder weisen die mehreren Windungen des Angioplastie-Katheters eine Biegeelastizität auf.

Vorzugsweise wird der Katheter nicht im aufgewickelten Zustand abgelegt, da er sich dann durch seine Biegeelastizität unkontrolliert strecken würde und dabei unsteril werden könnte.

Gemäß einer bevorzugten Weiterbildung hat die Vertiefung einen Rand oder zumindest einen Randabschnitt, an dem die eine oder die mehreren Windungen radial außen biegeelastisch abgestützt ist oder sind. Auf diese Weise ist der Angioplastie-Katheter mit seiner Eigenspannung oder Biegespannung in die Vertiefung gespannt und somit bereits ohne zusätzlich vorzusehende Stützpunkte oder Haltevorrichtungen, wie beispielsweise Halterungen oder Laschen oder Clips, am Träger gehalten.

Gemäß einer vorteilhaften Weiterbildung hat die Vertiefung wenigstens einen gesonderten Vertiefungsabschnitt oder Aufnahmeabschnitt, in dem einer der Endabschnitte, d.h., der Hub oder die Katheter-Spitze, aufgenommen ist. Dieser gesonderte Vertiefungsabschnitt oder Aufnahmeabschnitt ermöglicht ein verbessertes Halten des jeweiligen Endabschnitts am Träger aber auch ein präziseres Greifen des jeweiligen Endabschnitts durch den Operateur.

Gemäß einer Weiterbildung hat die Vertiefung mit Bezug zu dem Randabschnitt, insbesondere mit Bezug zu der einen oder zu den mehreren Windungen, einen etwa tangential auslaufenden ersten Aufnahmeabschnitt, in dem der Hub aufgenommen ist.

Vorzugsweise ist der erste Aufnahmeabschnitt mit einem Offset zu einer Außenkontur und/ oder Mantelfläche des Hubs ausgebildet, sodass der Hub darin in vorbestimmter oder definierter Lage aufgenommen und fest verankert ist und nicht mehr ungewollt herausrutschen kann.

Gemäß einer Weiterbildung hat die Vertiefung einen mit Bezug zu der einen Windung oder zu einer der mehreren Windungen tangential auslaufenden zweiten Aufnahmeabschnitt, in dem der distale Endabschnitt aufgenommen ist.

Gemäß einer weiteren vorteilhaften Weiterbildung der Erfindung ist der erste Aufnahmeabschnitt und/ oder der zweite Aufnahmeabschnitt des Trägers abknickbar ausgebildet, sodass der betreffende Aufnahmeabschnitt in der Weise vom Hub bzw. dem distalen Endabschnitt des Katheters weg geknickt oder weg gefaltet werden kann, dass der Hub und/ oder der distale Endabschnitt/ Ballon frei liegen und einfach gegriffen werden können.

Zur Ausbildung einer definierten Knicklinie oder Faltlinie kann eine durchgehende oder unterbrochene Schwächung oder Prägung am Träger vorgesehen sein. Bevorzugt ist die Schwächung, Knicklinie, Faltlinie oder Prägung linear.

Sofern der Träger nicht Teil der Sterilbarriere ist, kann eine durchgehende oder abschnittsweise Perforation des Trägers vorgesehen sein, um die Schwächung, Knicklinie oder Faltlinie auszubilden.

Die Schwächung oder Prägung verringert gemäß einer Weiterbildung die Dicke des Trägers, sodass dieser im Bereich der Schwächung oder Prägung ein geringeres Flächenträgheitsmoment aufweist und an dieser Stelle leichter geknickt bzw. gefaltet werden kann.

In einer Weiterbildung ist ein an dem Träger außenumfänglich umlaufender Rand vorgesehen, insbesondere um eine grundlegende Steifigkeit des Trägers gegen Knick zu erzielen.

Im Falle einer Weiterbildung mit dem am Träger außenumfänglich umlaufender Rand und mit der Knicklinie oder Faltlinie ist dieser Rand im Bereich der Knicklinie oder Faltlinie vorzugsweise unterbrochen oder ausgespart. So ist sichergestellt, dass die grundlegend versteifende Wirkung dieses Randes im Bereich der Knicklinie oder Faltlinie gezielt unterbrochen ist und das Knicken oder Falten gut möglich ist.

Alternativ kann anstelle der Knicklinie oder Faltlinie eine Abtrennlinie vorgesehen sein, entlang der der entsprechende Teil des Trägers abgetrennt, beispielsweise abgerissen werden kann. Da der abgerissene Teil aber separat entsorgt werden muss, was zusätzliche Arbeit bereitet, ist dies nicht die bevorzugte Ausführungsform.

Gemäß einer Weiterbildung unterteilt die wenigstens eine Schwächung, Prägung, Knicklinie, Faltlinie oder Perforierung den Träger in ein den ersten Aufnahmeabschnitt aufweisendes erstes Segment und/ oder in ein den zweiten Aufnahmeabschnitt aufweisendes zweites Segment und in ein die Windungen aufweisendes drittes Segment.

Gemäß einer Weiterbildung weist die Vertiefung eine Außenkontur auf, die den darin aufgenommenen Angioplastie-Katheter zumindest abschnittsweise berandet. Damit der Angioplastie-Katheter gut einlegbar und entnehmbar ist, ist vorzugsweise ein Offset der Außenkontur der Vertiefung zum Angioplastie-Katheter vorgesehen.

Vorzugsweise berandet die Außenkontur der Vertiefung den Angioplastie-Katheter entlang seiner kompletten Erstreckung, sodass der komplette Angioplastie-Katheter in der Vertiefung gehalten/ festgelegt ist.

In einer einfach zu fertigenden Weiterbildung, durch welche insbesondere bei der Herstellung der Bereitstellungsanordnung das Einlegen des Angioplastie-Katheters in die Vertiefung des Trägers einfach und gut automatisierbar ist, bildet der Träger mit seinem Rand oder Randabschnitt eine Schale aus.

Gemäß einer bevorzugten Weiterbildung läuft der Rand oder Randabschnitt, an dem die eine oder die mehreren Windungen radial außen biegeelastisch abgestützt ist oder sind, nur maximal einmal um, sodass vom Träger eine Schale gebildet ist. So können die Windungen gemeinsam als Bündel oder als Pulk radial außen am Rand oder Randabschnitt abgestützt sein. Das hat den Vorteil, dass der Angioplastie-Katheter mit seinen Windungen auf einfachste Weise "als Ganzes" gegriffen werden kann, bzw. umgekehrt, bei der Herstellung der Bereitstellungsanordnung "als Ganzes" in die Vertiefung eingelegt werden kann.

In einer anderen Weiterbildung läuft der Rand oder Randabschnitt, an dem die eine oder die mehreren Windungen radial außen biegeelastisch abgestützt ist oder sind, mehrfach um, insbesondere spiralförmig. So sind die Windungen jeweils einzeln abgestützt. Das heißt, an jedem Umlauf des Randes oder Randabschnitts ist je eine Windung abgestützt. Man kann auch sagen, der Angioplastie-Katheter ist Windung für Windung radial außen abgestützt.

In einer möglichen Ausbildung ist der spiralförmig umlaufende Randabschnitt von einer radial äußeren Flanke einer spiralförmig umlaufenden Nut gebildet.

Gemäß einer bevorzugten Weiterbildung ist der Randabschnitt, an dem die eine oder die mehreren Windungen radial außen biegeelastisch abgestützt ist oder sind, zumindest abschnittswiese derart steil und/ oder hinterschnitten ausgebildet, dass bei einem Greifen des Angioplastie-Katheters am Hub und einem Abziehen des Angioplastie-Katheters aus der Vertiefung, der Angioplastie-Katheter an dem Randabschnitt so geführt ist, dass ein Herausspringen der Windung oder der Windungen aus der Vertiefung verhindert ist.

Eine solche Ausgestaltung ermöglicht - ergänzend oder alternativ zum oben beschriebenen Greifen des Angioplastie-Katheters "als Ganzes" - das geführte Herausziehen des Angioplastie-Katheters aus dem Träger, so wie das der Operateur beispielsweise vom Stand der Technik gemäß dem spiralförmig aufgewickelten, röhrenförmigen Dispenser gewohnt ist.

Der Hinterschnitt kann vollständig umlaufend, insbesondere von einem vollständig umlaufenden Vorsprung, gebildet sein.

Alternativ oder ergänzend kann der Hinterschnitt abschnittsweise umlaufend, insbesondere von mehreren Vorsprüngen, gebildet sein.

Beide möglichen Ausprägungen des Hinterschnitts - vollständig umlaufend oder abschnittsweise umlaufend - verhindern jeweils das unbeabsichtigte Herausspringen des Angioplastie-Katheters, wenn dieser komplett in der Vertiefung ruht.

Der abschnittsweise umlaufende Hinterschnitt hat darüber hinaus den Vorteil, dass bei der Entnahme des Hubs oder der Spitze des Katheters aus der Vertiefung der restliche Katheter nur so weit aus der Vertiefung gleitet oder springt, bis er in Anlage an eine führende Kante ("leading edge") des nächsten Abschnitts des Hinterschnitts, insbesondere des nächsten Vorsprungs, der diesen Hinterschnitt ausbildet, kommt. An dieser Kante wird er dann durch seine nach außen wirksame Biegeelastizität selbsthemmend gestoppt. Für eine weitergehende Entnahme muss er wieder entgegen seiner nach außen wirksamen Biegeelastizität nach innen verformt werden, um diesen Vorsprung zu überwinden. Das kann aufgrund der Biegeelastizität nicht selbsttätig erfolgen, wodurch das ungewünschte selbsttätige Herausspringen des Angioplastie-Katheters verhindert ist. Stattdessen bedarf es des manuellen Eingriffs des Nutzers oder Operateurs, um den Angioplastie-Katheter um den Vorsprung herumzuführen und die Entnahme bis zum nächsten Vorsprung kontrolliert fortzuführen. Hier muss erneut manuell eingegriffen werden, und so weiter.

Gemäß einer bevorzugten Weiterbildung sind an dem radial äußeren Rand der Vertiefung, an dem die Windung oder Windungen des Angioplastie-Katheters abgestützt sind, für jede Windung des Angioplastie-Katheters wenigstens drei Vorsprünge vorgesehen. Die Vorsprünge sind dabei über einen Umfangswinkel der jeweiligen Windung verteilt angeordnet, wobei der Umfangswinkel größer als 180° ist, damit die oben beschriebene Selbsthemmung gegen unkontrolliertes Herausspringen oder -gleiten des Angioplastie-Katheters realisiert ist.

Gemäß einer bevorzugten Weiterbildung sind die Vorsprünge über den Umfangswinkel im Wesentlichen gleichverteilt, insbesondere mit gleichbleibendem Winkelabstand, angeordnet.

Gemäß einer bevorzugten Weiterbildung erstrecken sich die Vorsprünge in Radialrichtung nach innen, insbesondere in Richtung hin zu einem Zentrum oder mittigen Bereich des Trägers.

Gemäß einer bevorzugten Weiterbildung - insbesondere im Falle der als Bündel oder als Pulk radial außen am Rand oder Randabschnitt abgestützten Windungen - erstrecken sich die Vorsprünge zumindest abschnittsweise in Richtung eines Grundes oder Bodens der Vertiefung. Auf diese Weise ist der Angioplastie-Katheter noch sicherer in der Vertiefung gehalten.

Gemäß einer bevorzugten Weiterbildung - insbesondere im Falle der als Bündel oder als Pulk radial außen am Rand oder Randabschnitt abgestützten Windungen - erstrecken sich die Vorsprünge mindestens mit einem Bündel- oder Pulk-Durchmesser in Radialrichtung nach innen, vorzugsweise mindestens mit 125% bis 150% des Bündel- oder Pulk-Durchmessers. Das bewirkt, dass das Bündel oder der Pulk sicher in der Vertiefung gehalten ist.

Gemäß einer bevorzugten Weiterbildung - insbesondere im Falle des mehrfach umlaufenden Randes oder Randabschnitts der Vertiefung, insbesondere wenn die Windungen jeweils einzeln radial außen am Rand oder Randabschnitt abgestützt sind - erstrecken sich die Vorsprünge mindestens mit einem Viertel (25%) eines Durchmessers des Angioplastie-Katheters in Radialrichtung nach innen. Vorzugsweise erstrecken sie sich mit etwa 50%, maximal mit etwa 100% bis 150%, des Durchmessers des Angioplastie-Katheters in Radialrichtung nach innen. Über die derart auf den Durchmesser des Angioplastie-Katheters bezogene Erstreckung der Vorsprünge nach radial innen kann ein Optimum aus Abziehbarkeit des Angioplastie-Katheters aus der Vertiefung und Halten des Angioplastie-Katheters in der Vertiefung erzielt werden.

Gemäß einer bevorzugten Weiterbildung sind die Vorsprünge in Richtung eines Grundes oder Bodens der Vertiefung hakenförmig, insbesondere u-förmig oder v-förmig, gekrümmt. Auf diese Weise ist der Angioplastie-Katheter besonders sicher in der Vertiefung gehalten.

Eine Kombination aus dem vollständig umlaufendem und dem abschnittsweise umlaufendem Hinterschnitt (Vorsprünge) ist möglich, beispielsweise indem sich von dem vollständig umlaufenden Hinterschnitt wiederholt die Vorsprünge nach radial innen erstrecken.

Um die Greifbarkeit des Angioplastie-Katheters in der Vertiefung zu verbessern, ist gemäß einer bevorzugten Weiterbildung an dem Träger, insbesondere in einem Randbereich der Vertiefung, insbesondere in einem Bereich des Hubs und/ oder der Windung oder Windungen und/ oder des Katheter-Ballons, eine Greifmulde vorgesehen, die zumindest abschnittsweise vom aufgenommenen Angioplastie-Katheter, insbesondere vom Hub, von der Windung oder den Windungen oder von dem Katheter-Ballon, überspannt ist.

Alternativ oder ergänzend ist an dem Träger wenigstens eine Durchgangsausnehmung vorgesehen, die zumindest abschnittsweise vom aufgenommenen Angioplastie-Katheter, insbesondere vom Hub, von der Windung oder den Windungen oder von dem Katheter-Ballon, überspannt ist. Als Durchgangsausnehmung ist dabei ein Loch im Träger zu verstehen.

Alternativ kann man sagen, dass die Vertiefung von der Greifmulde oder der Durchgangsausnehmung unterbrochen ist. Unterbrochen bedeutet hierbei im Falle der Greifmulde, dass die Greifmulde tiefer als die Vertiefung ausgebildet ist.

Auf diese Weise kann an der Greifmulde oder der Durchgangsausnehmung der Hub, die eine oder die mehreren Windungen oder der den Ballon aufweisende Endabschnitt gut gegriffen/ untergriffen/ oder umgriffen werden und aus der Vertiefung angehoben oder entnommen und /oder abgezogen werden.

Vorzugsweise sind zwei, drei oder vier, Greifmulden oder Durchgangsausnehmungen entlang der einen oder der mehreren Windungen verteilt vorgesehen, vorzugsweise in gleichem Winkelabstand, um die o.g. Entnahme des Angioplastie-Katheters "als Ganzes" zu verbessern.

Gemäß einer bevorzugten Weiterbildung ist der Träger permeabel für ein Sterilisationsmedium und impermeabel für Keime vorgesehen und ausgestaltet. Selbstverständlich ist diese Weiterbildung nicht in der o.g. Ausführungsform zielführend, die die Durchgangsausnehmung zur Verbesserung der Greifbarkeit des Angioplastie-Katheters aufweist.

Vorzugsweise ist der Träger permeabel für Wasserdampf und/ oder Ethylenoxidgas, was im Falle der Ausgestaltung mit Greifmulde vorzugsweise durch eine Porosität des verwendeten Materials realisiert werden kann. Im Falle der Ausgestaltung mit der oben genannten Durchgangsausnehmung zur Verbesserung der Greifbarkeit ist eine solche Porosität nicht notwendig.

Gemäß einer bevorzugten Weiterbildung ist der Träger aus einer Folie, vorzugsweise einer Biofolie, tiefgezogen oder er ist aus einem Faserwerkstoff, vorzugsweise einem Biofaserwerkstoff, gegossen. Durch die Nutzung des genannten Bio-Materials wird beim Träger komplett auf Kunststoff verzichtet und die Menge anfallenden Kunststoff-Mülls ist reduziert. Die Nachhaltigkeit ist somit verbessert.

Möglich ist beispielsweise, eine polylactid-basierte, thermoformbare Folie (PLA-Folie) einzusetzen.

Gemäß einer Weiterbildung weist der Träger wenigstens eine Aussparung auf, wodurch ein Materialeinsatz und Gewicht des Trägers reduziert ist.

Vorzugsweise ist die Aussparung mittig am Träger, insbesondere radial innen oder konzentrisch mit Bezug zu der einen Windung oder den mehreren Windungen, vorgesehen.

Gemäß einer Weiterbildung ist die Vertiefung von einer mit dem Träger verbundenen Verschlussfolie überspannt, sodass der Angioplastie-Katheter von der Verschlussfolie in der Vertiefung gehalten ist, d.h., gegen ein ungewolltes Herausfallen oder -springen gesichert ist.

Gemäß einer bevorzugten Weiterbildung ist die Vertiefung von der Verschlussfolie keimdicht verschlossen. In diesem Fall bildet der Träger mit der Verschlussfolie eine Sterilbarriere aus. Voraussetzung für diese Weiterbildung ist, dass der Träger keine der oben beschriebenen Durchgangsausnehmungen zur Verbesserung der Greifbarkeit aufweist.

Gemäß einer alternativen Weiterbildung ist ein Verpackungsbeutel oder "Peelbeutel" vorgesehen, in dem der Träger unverschlossen angeordnet ist, wobei der Verpackungsbeutel keimdicht verschlossen ist. In diesem Fall dient der Träger somit nur dem Halten und späteren Ablegen/ Bereitstellen des Angioplastie-Katheters, wohingegen die Sterilbarriere von dem Verpackungsbeutel oder Peelbeutel ausgebildet wird.

Diese Weiterbildung mit Verschlussbeutel zur Ausbildung der Sterilbarriere ist insbesondere dann notwendig, wenn der Träger wenigstens einer der oben beschriebenen Durchgangsausnehmungen zum verbesserten Greifen des Angioplastie-Katheters aufweist.

Gemäß einer bevorzugten Weiterbildung ist auf der Verschlussfolie wenigstens eine Kenngröße des Angioplastie-Katheters, beispielsweise der Katheter-Typ, seine Artikelnummer, seine Charge, sein Herstellungsdatum, sein Verfalldatum, der Durchmesser des Führungsdrahtes, der Nenndurchmesser und die Länge des Ballons, der Nenndruck, der maximal zulässige Druck und/ oder das Druck-Durchmesser-Verhalten des Ballons abgebildet.

Damit die Verschlussfolie am Träger verbleibt und insbesondere die o.g. Informationen zur wenigstens einen Kenngröße nicht verloren gehen, wenn die Verschlussfolie geöffnet wurde, ist ein Öffnungsabschnitt der Verschlussfolie mit dem Träger lösbar verbunden, wohingegen ein Verbindungsabschnitt der Verschlussfolie fester als der Öffnungsabschnitt mit dem Träger verbunden ist, insbesondere um am Träger zu verbleiben. Bei geöffneter Bereitstellungsanordnung liegt so der Angioplastie-Katheter offen in der Vertiefung des Trägers bereit und die Kennwerte sind trotzdem am Träger gegen Verlust gesichert verfügbar. Somit ist eine Spezifikation des Angioplastie-Katheters immer präsent.

Gemäß einer bevorzugten Weiterbildung sind der Verbindungsabschnitt und der Öffnungsabschnitt mit dem Träger geklebt.

Gemäß einer Weiterbildung sind der Verbindungsabschnitt und der Öffnungsabschnitt mit unterschiedlich ausgebildeten Klebschichten mit dem Träger verbunden, um die festere Verbindung des Verbindungsabschnitts mit dem Träger zu erzielen.

Gemäß einer Weiterbildung sind der Verbindungsabschnitt und der Öffnungsabschnitt mit Bezug zum Träger beim Kleben mit unterschiedlichen Prozessparametern beaufschlagt, um die festere Verbindung des Verbindungsabschnitts mit dem Träger zu erzielen. Insbesondere sind die Prozessparameter beim Kleben: eine vorbereitende Oberflächenbehandlung des Verbindungsabschnitts, Öffnungsabschnitts und/ oder Trägers, eine Art und eine Auftragsart des Klebstoffs, eine Temperatur und ein Druck beim Auftragen und/ oder Aushärten, eine Aushärtezeit und/ oder eine Fixierungszeit.

Gemäß einer anderen bevorzugten Weiterbildung sind der Verbindungsabschnitt und der Öffnungsabschnitt mit dem Träger versiegelt oder verschweißt.

Gemäß einer Weiterbildung sind der Verbindungsabschnitt und der Öffnungsabschnitt mit Bezug zum Träger beim Siegeln oder Schweißen mit unterschiedlichen Prozessparametern beaufschlagt, um die festere Verbindung des Verbindungsabschnitts mit dem Träger zu erzielen. Insbesondere sind die Prozessparameter beim Siegeln oder Schweißen: eine Erwärmungszeit, eine Temperatur und/ oder ein Anpressdruck des Verbindungsabschnitts, Öffnungsabschnitts und/ oder Trägers.

Der Angioplastie-Katheter kann dann nach dem Teilabzug der Verschlussfolie für die Angioplastie vorbereitet werden, ohne dass er dazu entnommen werden muss. Der Träger kann nach dem Teilabzug der Verschlussfolie auch für eine Zwischenaufbewahrung des Angioplastie-Katheters wiederverwendet werden.

Ein Verfahren zur Herstellung einer Bereitstellungsanordnung für eine perkutane transluminale Angioplastie, insbesondere zur Herstellung einer

Bereitstellungsanordnung, die gemäß wenigstens einem Aspekt der vorhergehenden Beschreibung ausgestaltet ist, hat offenbarungsgemäß folgende Schritte:
- Urformen oder Umformen eines flächigen Trägers mit einer Vertiefung; und
- Einlegen eines Angioplastie-Katheters, der an einem vorbestimmt proximalen Endabschnitt einen Hub und an einem vorbestimmt distalen Endabschnitt wenigstens einen Katheter-Ballon aufweist, in die Vertiefung derart, dass der Angioplastie-Katheter in einer oder in mehreren Windungen in der Vertiefung aufgenommen und gehalten ist.

Mittels dem Urformen oder Umformen kann der flächige Träger mit der Vertiefung bei geringen Kosten gefertigt werden. Das Urformen oder Umformen ermöglicht zudem eine nahezu unbegrenzte Varianz in der Formgebung der Vertiefung. So kann die Vertiefung auf kostengünstige Weise so gefertigt werden, dass der Angioplastie-Katheter auf einfachste Weise in die Vertiefung eingelegt werden kann und dort sicher gehalten werden kann. So ist ein Verfahren zur Herstellung der Bereitstellungsanordnung geschaffen, das erlaubt, den Aufwand zur Automatisierung gering zu halten oder zu verringern.

Gemäß einer Weiterbildung des Verfahrens erfolgt das Umformen durch Tiefziehen einer Folie, vorzugsweise einer Biofolie, und das Urformen durch Faserguss eines Faserwerkstoffs, vorzugsweise eines Bio-Faserwerkstoffs. Die Nachhaltigkeit ist somit erhöht. Nicht-Faserwerkstoffe können spritzgegossen werden.

In einer Variante des Verfahrens sind folgende Schritte vorgesehen:
- Überspannen der Vertiefung des Trägers mit einer Verschlussfolie; und
- Verbinden der Verschlussfolie mit dem Träger, beispielsweise durch Siegeln (Heißsiegeln), Kleben, Schmelzschweißen (inkl. Ultraschallschweißen, Laserschweißen und Heizelementstumpfschweißen) oder Pressschweißen. Auf diese Weise ist der Angioplastie-Katheter in einem vom Träger und der Verschlussfolie begrenzten Aufnahmeraum keimdicht verpackt. Der Träger bildet somit zusammen mit der Verschlussfolie die Sterilbarriere aus.

Alternativ oder ergänzend zu den Schritten Überspannen der Vertiefung des Trägers mit einer Verschlussfolie und Verbinden der Verschlussfolie mit dem Träger hat das Verfahren folgende Schritte:
- Packen des Trägers in einen Verschlussbeutel; und
- Verschließen des Verschlussbeutels, beispielsweise durch eine der im vorstehenden Absatz genannten Verbindungstechniken. Auf diese Weise sind der Träger und der daran gehaltene Katheter keimdicht im Verschlussbeutel verpackt, ohne dass die Vertiefung extra mit einer Verschlussfolie verschlossen sein muss.

Diese beiden letztgenannten Schritte sind insbesondere notwendig, wenn der Träger gar nicht geeignet ist, eine Sterilbarriere auszubilden. Das ist beispielsweise dann der Fall, wenn er wenigstens eine der weiter oben beschriebenen Durchgangsausnehmungen zum verbesserten Greifen des Angioplastie-Katheters aufweist.

Jede Offenbarung im Zusammenhang mit der Bereitstellungsanordnung gemäß der vorliegenden Offenbarung gilt für das Verfahren gemäß der vorliegenden Offenbarung, und umgekehrt.

Die Offenbarung wird nachfolgend mit Hilfe von Zeichnungen weiter erläutert. Es zeigen:
- Fig. 1: eine Draufsicht auf eine erste Ausführungsform einer offenbarungsgemäßen Bereitstellungsanordnung mit einem Träger, einem am Träger aufgenommenen Angioplastie-Katheter und mit einer halb abgezogenen Verschlussfolie;
- Fig. 2: eine Rückansicht der Bereitstellungsanordnung gemäß Figur 1;
- Fig. 3: eine Draufsicht auf eine zweite Ausführungsform einer offenbarungsgemäßen Bereitstellungsanordnung mit einem Träger und einem am Träger aufgenommenen Angioplastie-Katheter bei komplett abgezogener Verschlussfolie;
- Fig. 4: eine Rückansicht auf eine dritte Ausführungsform einer offenbarungsgemäßen Bereitstellungsanordnung mit einem Träger, einem am Träger aufgenommenen Angioplastie-Katheter und mit einer Verschlussfolie;
- Fig. 5: einen Schnitt der Bereitstellungsanordnung gemäß Figur 1;
- Fig. 6: einen Schnitt einer vierten Ausführungsform einer offenbarungsgemäßen Bereitstellungsanordnung;
- Fig. 7: einen Schnitt einer fünften Ausführungsform einer offenbarungsgemäßen Bereitstellungsanordnung; und
- Fig. 8: ein Ablaufdiagramm eines Verfahrens zur Herstellung einer offenbarungsgemäßen Bereitstellungsanordnung gemäß einer Ausführungsform.

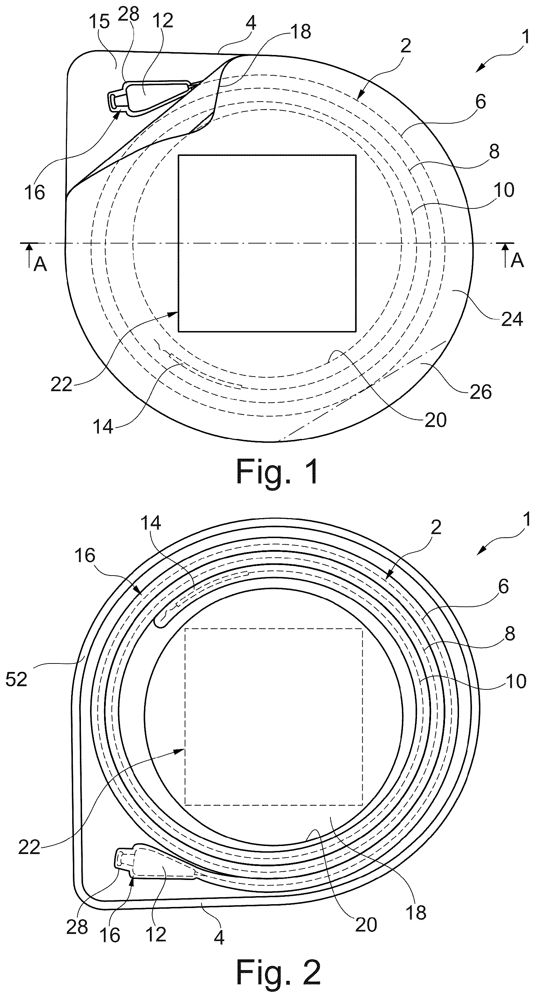

Figur 1 zeigt eine Bereitstellungsanordnung 1 für die perkutane transluminale Angioplastie gemäß einer ersten Ausführungsform in einer Draufsicht, wie sie sich einem Operateur beim Öffnen der Bereitstellungsanordnung 1 darstellt.

Die Bereitstellungsanordnung 1 hat einen überwiegend kreisförmigen, flächigen Träger 4, an dem einseitig ein steriler Angioplastie-Katheter 2 angeordnet und gehalten ist. Der Angioplastie-Katheter 2 ist an dem Träger 4 in mehreren Windungen 6, 8, 10 aufgerollt gehalten. An seinem vorbestimmt proximalen Endabschnitt hat der Angioplastie-Katheter 2 einen Hub 12 oder Anschluss, insbesondere zum Anschluss einer Angioplastie-Spritze, und an seinem vorbestimmt distalen Endabschnitt hat er einen Katheter-Ballon 14.

Der Träger 4 hat an seiner Flachseite 15 eine Vertiefung 16, in der der komplette Angioplastie-Katheter 2 aufgenommen und gehalten ist. Die Vertiefung 16 hat einen mit Bezug zu der radial äußersten Windung 6 tangential auslaufenden, ersten Aufnahmeabschnitt 28 oder Halteabschnitt, in dem der Hub 12 aufgenommen ist. Der erste Aufnahmeabschnitt 28 weist dabei einen Offset zu einer Außenkontur des Hub 12 auf, der so bemessen ist, dass der Hub 12 einerseits gut in den ersten Aufnahmeabschnitt 28 einlegbar ist, gegen Verrutschen oder Herausrutschen gehalten ist und dennoch gut greifbar und entnehmbar ist. Im Bereich des ersten Aufnahmeabschnitts 28 weist der Träger 4 einen seine Kreisform unterbrechenden Eckabschnitt auf.

Gemäß Figur 1 ist die Vertiefung 16 von einer Verschlussfolie 18 überspannt. Die Verschlussfolie 18 ist mit der Flachseite 15 des Trägers 4 stoffschlüssig verbunden, insbesondere heißversiegelt.

Die Vertiefung 16 gemäß Figur 1 ist spiralförmig umlaufend ausgestaltet, wobei in der Darstellung gemäß Figur 1 auf eine Darstellung der von der Verschlussfolie 18 verdeckten Spiralarme der Vertiefung 16 verzichtet wird, um die Figur 1 nicht zu überfrachten. In der spiralförmig umlaufenden Vertiefung 16 verlaufen die Windungen 6, 8, 10 ausgehend vom Hub 12 hin zu dem Katheter-Ballon 14. Beides, sowohl die Windungen 6, 8, 10 als auch der Katheter-Ballon 14, sind in Figur 1 gestrichelt dargestellt, da sie von der Verschlussfolie 18 verdeckt sind.

Die Verschlussfolie 18 ist in der Darstellung gemäß Figur 1 gerade so weit von der Flachseite 15 des Trägers 4 abgezogen, dass der Hub 12 freigelegt ist, was den Moment darstellen soll, in dem die Bereitstellungsanordnung 1 in Vorbereitung oder in Durchführung der Angioplastie vom Operateur für die Entnahme des Angioplastie-Katheters 2 geöffnet wird.

Im verschlossenen Zustand der Bereitstellungsanordnung 1 ist die Verschlussfolie 18 an der Flachseite 15 vollumfänglich oder vollflächig mit dem Träger 4 verbunden. Die Verschlussfolie 18 bildet dann zusammen mit dem Träger 4 die Sterilbarriere für den in der Vertiefung 16 gehaltenen Angioplastie-Katheter 2 aus. Die Vertiefung 16 repräsentiert dabei einen sterilen Aufnahmeraum des Angioplastie-Katheters 2.

Über den Träger 4 und die Verschlussfolie 18 der Bereitstellungsanordnung 1 gemäß Figur 1 ist somit mit einem geringen Materialeinsatz eine kompakte Sterilbarriere für den Angioplastie-Katheter 2 ausgebildet.

Auf der dem Operateur zugewandten Oberseite oder Außenseite der Verschlussfolie 18 ist ein Datenfeld 22 mit allen relevanten Kenngrößen des Angioplastie-Katheters 2 abgebildet. Die Kenngrößen sind der beinhaltete Katheter-Typ, seine Artikelnummer und Charge, sein Herstellungsdatum und Verfalldatum, ein Durchmesser des Führungsdrahtes, ein Nenndurchmesser und eine Länge des Ballons, ein Nenndruck und ein maximal zulässiger Druck und ein Druck-Durchmesser-Verhalten des Ballons, das sogenannte Compliance Chart.

In einem Bereich diametral zu dem Eckbereich der Flachseite 15, also diametral zum ersten Aufnahmeabschnitt 28, bzw. Hub 12, hat die Verschlussfolie 18 einen Verbindungsabschnitt 26, der so fest mit dem Träger 4 stoffschlüssig verbunden ist, dass sichergestellt ist, dass die oben genannten Kenngrößen und Informationen zum Angioplastie-Katheter 2 nicht verloren gehen und verfügbar bleiben, selbst wenn die Verschlussfolie 18 geöffnet wird. Ein diametral zum Verbindungsabschnitt 26 vorgesehener Öffnungsabschnitt 24 der Verschlussfolie 18 erstreckt sich gemäß Figur 1 ausgehend vom Verbindungsabschnitt 26 bis hin zu dem Eckbereich der Flachseite 15.

Der Öffnungsabschnitt 24 ist verglichen mit dem Verbindungsabschnitt 26 weniger fest mit dem Träger 4 verbunden, damit er mit angemessenem Kraftaufwand vom Operateur abzuziehen ist.

Der Öffnungsabschnitt 24 ist so bemessen, dass er den kompletten Angioplastie-Katheter 2 überspannt, sodass beim Abziehen des Öffnungsabschnitts 24 der Hub 12, alle Windungen 6, 8, 10 und die Katheter-Spitze 14 freigelegt werden, bis der fester verbundene Verbindungsabschnitt 26 das Abziehen sprunghaft erschwert und der Operateur das Abziehen intuitiv stoppen kann.

Die oben genannte, festere Verbindung des Verbindungsabschnitts 26 ist nur so fest ausgelegt, dass der Verbindungsabschnitt 26 für eine spätere Mülltrennung manuell oder automatisiert abgezogen werden kann.

Der Angioplastie-Katheter 2 kann dann nach dem Teilabzug der Verschlussfolie 18 bis zum Verbindungsabschnitt 26 für die Angioplastie vorbereitet werden, ohne dass er dazu aus dem Träger 4, genauer gesagt aus dessen Vertiefung 16, entnommen werden muss. Der Träger 4 kann nach dem Teilabzug der Verschlussfolie 18 zudem für eine Zwischenaufbewahrung des Angioplastie-Katheters 2 wiederverwendet werden.

Der Träger 4 weist mittig, das heißt etwa konzentrisch zu den Windungen 6, 8, 10 eine kreisförmige Durchgangs-Ausnehmung oder Durchgangs-Aussparung 20 auf, um so den Materialverbrauch für die Bereitstellungsanordnung 1 klein zu halten.

Figur 2 zeigt eine Rückansicht, bzw. Unteransicht, der Bereitstellungsanordnung 1 gemäß Figur 1. Gut zu erkennen ist der flächige Charakter des Trägers 4 daran, dass sich die oben beschriebene Vertiefung 16 in der Flachseite 15 des Trägers 4 dem Betrachter entgegenwölbt. Wie vorbeschrieben erstreckt sich der (gestrichelt dargestellte) Angioplastie-Katheter 2 aus dem ersten Aufnahmeabschnitt 28 der Vertiefung 16 mit seiner radial äußeren Windung 6, seiner radial mittleren Windung 8 und seiner radial inneren Windung 10 bis zu seinem distalen Endabschnitt mit dem Ballon-Katheter 14.

Gemäß dem ersten Ausführungsbeispiel gemäß den Figuren 1 und 2 erstreckt sich die Vertiefung 16 ausgehend vom ersten Aufnahmeabschnitt 28 als spiralförmige Nut.

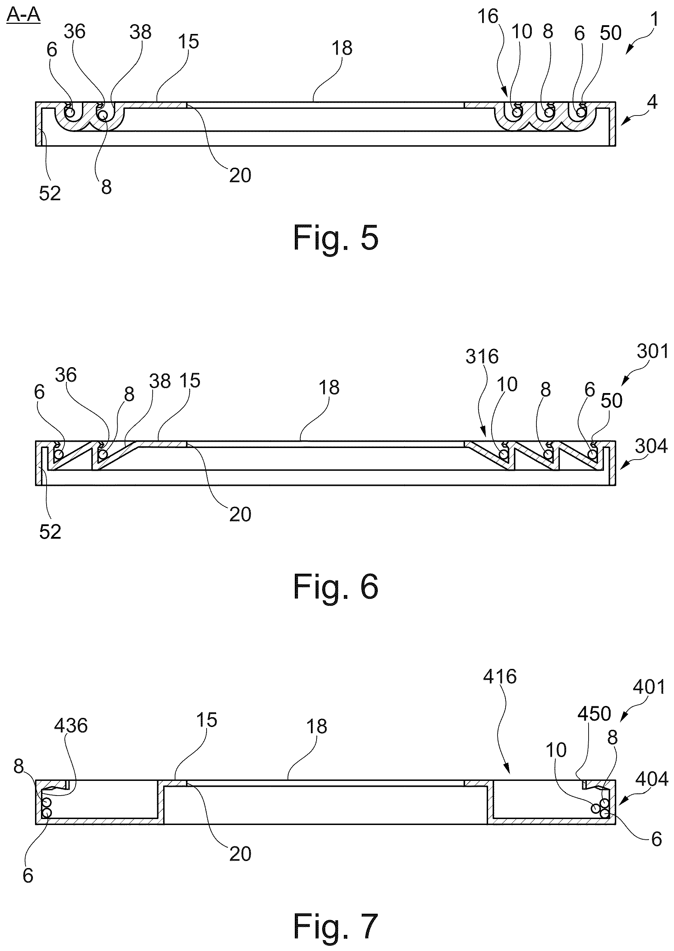

Zum Schutz gegen Knick weist der Träger 4 einen rückwärtig umlaufenden Rand 52 auf (vgl. auch Figur 5). Der Rand 52 ist rückwärtig ausgebildet, um den Verschluss oder die Versiegelung der vorderseitigen Vertiefung 16 mit der Verschlussfolie 18 und die Entnahme des Angioplastie-Katheters 2 aus der Vertiefung 16 nicht zu erschweren.

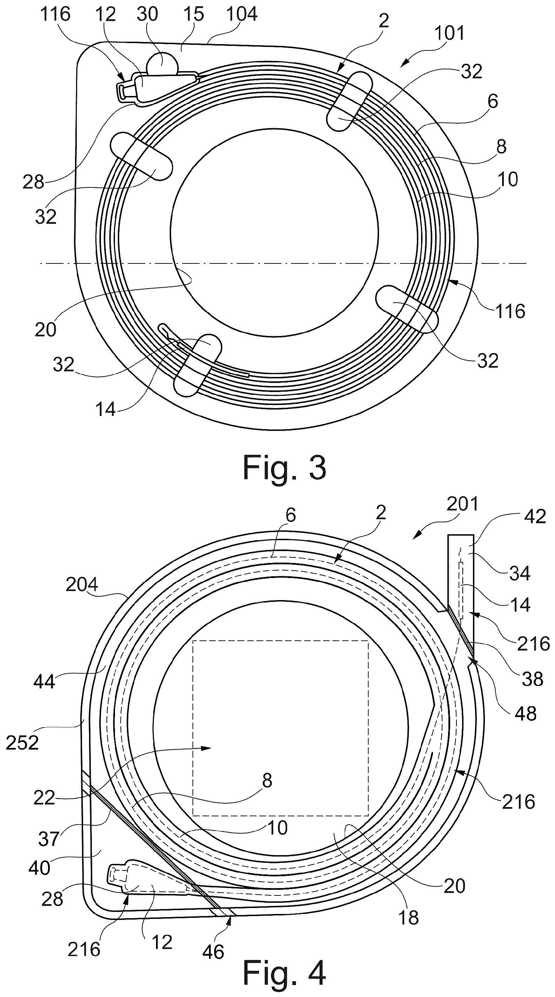

Figur 3 zeigt eine Draufsicht auf eine zweite Ausführungsform einer offenbarungsgemäßen Bereitstellungsanordnung 101 mit einem Träger 104 und dem am Träger 104 gehaltenen Angioplastie-Katheter 2 bei komplett abgezogener Verschlussfolie.

Um die Beschreibung nicht zu überfrachten, wird im Folgenden stets nur auf die Unterschiede zur ersten Ausführungsform gemäß den Figuren 1 und 2 eingegangen. Über die Ausführungsformen gleichbleibende Komponenten sind zudem mit den gleichen Bezugszeichen versehen.

Gemäß Figur 3 sind am Träger 104, genauer gesagt in dessen Flachseite 15, mehrere Greifmulden 30, 32 vorgesehen, die jeweils in die Vertiefung 116 münden. Die Greifmulde 30 mündet lateral in den ersten Aufnahmeabschnitt 28, sodass der Hub 12 mit einem Finger lateral untergreifbar ist und aus dem ersten Aufnahmeabschnitt 28 entnehmbar ist. Die weiteren vier Greifmulden 32 sind mit gleichem Winkelabstand entlang den Windungen 6, 8, 10 verteilt angeordnet, wobei sie in der Flachseite 15 tiefer als die spiralförmige Vertiefung 116 ausgebildet sind und somit die Vertiefung 116 unterbrechen. Somit überspannen die Windungen 6, 8, 10 die jeweilige Greifmulde 32. So können die Windungen 6, 8, 10 von radial innen und von radial außen gemeinsam gegriffen werden und der Angioplastie-Katheter 2 kann "in Einem" oder "als Ganzes" von dem Träger 104 entnommen werden.

Bei einer alternativen - nicht gezeigten - Ausführungsform einer offenbarungsgemäßen Bereitstellungsanordnung ist der Zugriff auf den Angioplastie-Katheter, anstatt mittels Greifmulden, durch wenigstens eine Durchgangsausnehmung realisiert, die im Prinzip ein Loch im Träger darstellt. Wichtig hierbei ist zu beachten, dass der so perforierte Träger dann keinen Beitrag mehr zu einer Sterilbarriere leisten kann. Er ist dann allein auf seine Träger- und Haltefunktion beschränkt. In dieser Ausführungsform ist dann zur Ausbildung der Sterilbarriere eine zusätzliche Verpackung notwendig, beispielsweise, indem der Träger mit dem daran gehaltenen Angioplastie-Katheter in einem Verschlussbeutel ("Peelbeutel") angeordnet ist, der impermeabel für Keime ist.

Figur 4 zeigt eine Rückansicht einer dritten Ausführungsform einer offenbarungsgemäßen Bereitstellungsanordnung 201 mit einem Träger 204, mit dem am Träger 204 aufgenommenen Angioplastie-Katheter 2 und mit einer Verschlussfolie 18.

In einem Bereich des Trägers 204, der etwa diametral zum ersten Aufnahmeabschnitt 28 angeordnet ist, bildet die Vertiefung 216 an dem Träger 204 eine längliche Ausstülpung/ einen länglichen Fortsatz und damit einen zweiten Aufnahmeabschnitt 34, in dem der Katheter-Ballon 14 gesondert von den restlichen Windungen 6, 8, 10 vorliegt und damit bei abgezogener Verschlussfolie 18 gesondert greifbar ist. Auf diese Weise kann die Katheter-Spitze/ der Katheter-Ballon 14 vom Operateur gesondert gegriffen werden und in Vorbereitung oder Durchführung der Angioplastie in die Schleuse am arteriellen Zugang des Patienten eingeführt werden, ohne dass dazu die restlichen Bestandteile des Angioplastie-Katheters 2, also die Windungen 6, 8, 10 und der Hub 12, entnommen werden müssen. So kann während der Einführung der Katheter-Spitze der überwiegende und längste Teil des Angioplastie-Katheters 2 geordnet und sicher im Träger 204 verwahrt bleiben, was den Operateur im Handling entlastet.

Um dem Operateur das Greifen des Hubs 12 und der Katheter-Spitze/ des Katheter-Ballons 14 noch einfacher zu machen, sind an dem Träger 204 zwei lineare, jeweils von einer Prägung gebildete Schwächungen 37, 38 vorgesehen, die als Knick- oder Faltlinie dienen. Durch sie ist das dortige Flächenträgheitsmoment des Trägers 204 so geschwächt, dass ein Knick-/ Faltwiderstand geringer ist.

Durch diese Prägungen 37, 38 ist der Träger 204 in drei relativ zueinander weg knickbare/ weg faltbare Segmente 40, 42 und 44 unterteilt. Dadurch können der erste Aufnahmeabschnitt 28 und der zweite Aufnahmeabschnitt 34 wahlweise vom mittigen, dritten Segment des Trägers 204 weg geknickt werden, wodurch wahlweise der Hub 12 und/ oder der Katheter-Ballon 14 frei gelegt ist und völlig ungehindert gegriffen werden kann.

Die Prägungen 37, 38 verlaufen im Ausführungsbeispiel gemäß Figur 4 linear. Sie können alternativ ganz oder abschnittsweise gekrümmt verlaufen, beispielsweise angepasst an den gekrümmten Verlauf der Vertiefung 216 im Bereich der jeweils benachbarten Windung 8, bzw. 6.

Auch der Träger 204 des Ausführungsbeispiels gemäß Figur 4 weist einen außenumfänglich umlaufenden Rand 252 auf. Abweichend vom Rand 52 des Ausführungsbeispiels gemäß Figur 1 und 2 ist der Rand 252 gemäß Figur 4 in den Bereichen der Prägungen 37, 38 jeweils unterbrochen. Der Rand 252 hat in diesen Bereichen jeweils eine Aussparung 46, 48, damit dort seine versteifende Wirkung gezielt unterbrochen ist. So wird das Wegknicken oder Wegfalten des jeweiligen Segmentes 40, 42 vom mittigen, dritten Segment 44 gezielt ermöglicht und erleichtert, wobei der Rand 252, abgesehen von den Aussparungen 46, 48, weiterhin seine versteifende Wirkung erfüllt.

Figur 5 zeigt einen Schnitt A-A der Bereitstellungsanordnung 1 gemäß Figur 1. Dementsprechend ist die spiralförmig umlaufende Vertiefung 16 in Figur 5 rechts im Bereich der drei Windungen 6, 8, 10 und in Figur 5 links lediglich im Bereich der radial äußeren Windung 6 und der radial mittleren Windung 8 geschnitten. Die Vertiefung 16 ist als Nut mit verrundetem Nutgrund ausgebildet.

Beide Flanken oder Ränder der Nut, d. h., ein radial äußerer Rand oder Randabschnitt 36 der Vertiefung 16 und ein radial innerer Rand oder Randabschnitt 38 der Vertiefung 16, sind mit Bezug zu der Flachseite 15 so steil ausgebildet, dass sich die Nut/ die Vertiefung 16 hin zur Flachseite 15 wieder etwas verjüngt. Auf diese Weise ist insbesondere vom radial äußeren Rand oder Randabschnitt 36 ein vollständig umlaufender Hinterschnitt an der Vertiefung 16 ausgebildet, durch den die Windungen 6, 8, 10 grundlegend gegen ein Herausspringen oder Herausgleiten aus der Nut/ der Vertiefung 16 gesichert sind.

Ergänzend zu dem vollständig umlaufenden Hinterschitt ist ein lediglich abschnittsweise umlaufender Hinterschnitt vorgesehen, der von vereinzelten Vorsprüngen 50 gebildet ist, die in regelmäßigem Abstand entlang dem radial äußeren Rand oder Randabschnitt 36 wiederholt angeordnet sind. Im gezeigten Ausführungsbeispiel sind pro Windung 6, 8 vier und für die Windung 10 drei derartige Vorsprünge 50 vorgesehen, von denen aufgrund der Schnittführung in Figur 5 pro Windung 6, 8 lediglich zwei und pro Windung 10 nur eine sichtbar sind. Die Vorsprünge 50 sind über den Umfangswinkel der jeweiligen Windung 6, 8, 10 im Wesentlichen gleichverteilt angeordnet.

Entnimmt nun der Nutzer beispielsweise den Ballon 14 des Angioplastie-Katheters 2 aus der Vertiefung 16 gemäß Figur 1, ist der o.g. komplett umlaufende Hinterschnitt des Randes 36 allein durch diese Entnahme bereits überwunden und der Katheter 2 beginnt aufgrund seiner nach außen wirksamen Biegeelastizität entlang diesem Hinterschnitt abzugleiten. Dabei gleitet der Angioplastie-Katheter 2 aber nur so weit aus der Vertiefung 16, bis er in Anlage an die führende Kante ("leading edge") eines der Vorsprünge 50 kommt. Auch hier wirkt die Biegeelastizität nach radial außen und es kommt so zur Selbsthemmung des Angioplastie-Katheters 2 an dem betreffenden Vorsprung 50. Verformt (im Sinne von elastischer Verformung) der Nutzer den Angioplastie-Katheter 2 nun an dieser Stelle wieder nach radial innen, kann der betreffende Vorsprung 50 überwunden werden und die Entnahme kann kontrolliert fortgesetzt werden bis zum nächsten Vorsprung 50, und so weiter. Der Begriff des Verformens bedeutet hierbei, dass der aus dem Träger hervorstehende Teil des Katheters zur Mitte des Trägers hin bewegt wird, wobei der Katheter teilweise elastisch verformt wird.

Ein weiterer Vorteil des vollständig umlaufenden Hinterschnitts und der Vorsprünge 50 ist, dass der Angioplastie-Katheter 2 an dem Hub 12 (vgl. Figur 1) in einer Richtung etwa tangential zur radial äußeren Windung 6 so aus dem Träger 4 herausgezogen werden kann, dass er beim Herausziehen bis zum Schluss in der Nut/ der Vertiefung 16 geführt verbleibt und nicht ungewollt aus der Nut/ der Vertiefung 16 herausspringt.

Figur 6 zeigt einen Schnitt einer vierten Ausführungsform einer offenbarungsgemäßen Bereitstellungsanordnung 301. Abweichend von der vorbeschriebenen Ausführungsform gemäß den Figuren 1, 2 und 5 ist hierbei die Vertiefung 316 als Nut mit stark unsymmetrischem Querschnitt ausgebildet. Dabei ist der radial äußere Randabschnitt 36 der Vertiefung 316 gleich demjenigen gemäß Figur 5 ausgebildet, das heißt, steil und mit dem vorbeschriebenen vollständig umlaufenden Hinterschnitt, sowie mit den Vorsprüngen 50. Ein radial innerer Randabschnitt 38 der Vertiefung 316, bzw. die radial innere Flanke der Nut 316 ist verglichen dazu flach ausgebildet. Auf diese Weise kann bei der Herstellung der Bereitstellungsanordnung 301 gemäß Figur 6 der Angioplastie-Katheter 2 einfacher in die Vertiefung 316 eingelegt werden, sei es manuell oder automatisiert.

Aufgrund der Elastizität der Windungen 6, 8, 10 ist der Angioplastie-Katheter 2, wie auch in den vorbeschriebenen Ausführungsformen, nach radial außen gegen den Randabschnitt 36 gespannt und dort radial abgestützt. Diese Abstützung zusammen mit dem genannten vollständig umlaufenden Hinterschnitt und den Vorsprüngen 50 am radial äußeren Randabschnitt 36 führt auch bei der Ausführungsform gemäß Figur 6 dazu, dass der Angioplastie-Katheter 2 beim Abziehen aus der Vertiefung 316 gegen Herausspringen geschützt ist. Die Wirkung und Vorteile der Vorsprünge 50 sind bei der Bereitstellungsanordnung 301 die gleichen, wie bei der Bereitstellungsanordnung 1 gemäß den Figuren 1, 2, 5.

Figur 7 zeigt einen Schnitt einer fünften Ausführungsform einer offenbarungsgemäßen Bereitstellungsanordnung 401. Abweichend von allen vorgenannten Ausführungsformen ist hierbei die Vertiefung 416 nicht spiralförmig umlaufend ausgestaltet, sondern sie läuft maximal einmal um. Sie ist abgesehen vom oben beschriebenen Eckbereich kreisringförmig umlaufend ausgebildet, sodass alle Windungen 6, 8, 10 an dem radial außen angeordneten Randabschnitt 436 gemeinsam, sozusagen als "Pulk" oder "Bündel", abgestützt sind. Die Vertiefung 416 ist damit in radialer Richtung sehr breit was bei der Herstellung der Bereitstellungsanordnung 401 das Einlegen des Angioplastie-Katheters 2, sei es manuell oder automatisiert, noch einmal vereinfacht. Auch in dieser Ausführungsform ist der radial äußere Randabschnitt 436 mit dem vorbeschriebenen, vollumfänglichen Hinterschnitt und mit Vorsprüngen 450 ausgeführt, um das vorbeschriebene Abziehen des Angioplastie-Katheters 2 ohne Herausspringen zu ermöglichen.

Verglichen mit den Vorsprüngen 50 gemäß den Figuren 5 und 6 sind die Vorsprünge 252 gemäß Figur 7 in Form eines Hakens, einer Kralle oder einer Harke ausgebildet und kragen bedeutend weiter und mit mehr Hinterschnitt nach radial innen in die Vertiefung 416 hinein. Das ist dem Umstand geschuldet, dass die im Pulk angeordneten Windungen 6, 8, 10 nicht vereinzelt geführt sind, wie das beispielsweise für die vereinzelt und spiralförmig geführten Windungen 6, 8, 10 gemäß den Figuren 1 bis 6 der Fall ist. Beim Abziehen oder Abheben beispielsweise des Hubs 12 aus der Vertiefung 416 können sich daher die Windungen 6, 8, 10 ggf. in geringem Maße gegenseitig hemmen und mitnehmen. Die weit auskragenden Vorsprünge/ Harken 252 verhindern dann beispielsweise, dass eine Windung, die jeweils andere ungewollt mit abhebt und mit aus der Vertiefung 416 zieht.

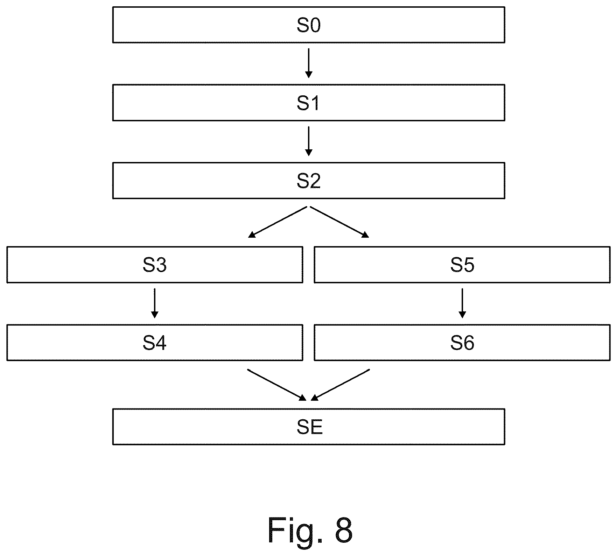

Figur 8 zeigt ein Ablaufdiagramm eines offenbarungsgemäßen Verfahrens zur Herstellung einer offenbarungsgemäßen Bereitstellungsanordnung, insbesondere einer Bereitstellungsanordnung, die gemäß wenigstens einem Aspekt der vorangegangenen Beschreibung ausgestaltet ist.

Hierbei erfolgt zunächst ein Schritt S0 Initialisieren oder Starten der Herstellung. Die weitere Beschreibung erfolgt zur Veranschaulichung unter Bezugnahme auf Figur 1, 2 und 5, die die erste Ausführungsform der hergestellten Bereitstellungsanordnung 1 zeigen.

Nach dem Schritt S0 Initialisieren folgt ein Schritt Urformen und/oder Umformen S1 des flächigen Trägers 4 mit der Vertiefung 16. Dabei kann das Urformen und/oder Umformen durch Tiefziehen einer Folie, vorzugsweise einer Biofolie, oder durch Fasergießen eines Faserwerkstoffs, vorzugsweise eines Bio-Faserwerkstoffs, erfolgen. Mit derartigen Bio-Folien oder Bio-Werkstoffen ist die Nachhaltigkeit erhöht. Mit Nicht-Faserwerkstoffen kann ein Spritzgießen erfolgen.

Es folgt das manuelle oder vorzugsweise automatisierte Einlegen S2 des Angioplastie-Katheters 2 in die Vertiefung 16 derart, dass der Angioplastie-Katheter 2 in mehreren Windungen 6, 8, 10 in der Vertiefung 16 aufgenommen und gehalten ist. Das Halten resultiert dabei zum einen aus der Formgebung der Vertiefung 16, die wie vorbeschrieben den radial äußeren Randabschnitt 36 mit Hinterschnitt und den ersten Aufnahmeabschnitt 28 hat, und zum anderen aus der Elastizität der Windungen 6, 8, 10, aufgrund dessen der Angioplastie-Katheter 2 biegeelastisch gegen den radial äußeren Randabschnitt 36 gespannt ist.

Um den Angioplastie-Katheter 2 steril zu verpacken und eine Sterilbarriere auszubilden, folgen in einer Variante des Verfahrens Schritte Überspannen S3 der Vertiefung 16 mit einer Verschlussfolie 18 und Verbinden S4 der Verschlussfolie 18 mit dem Träger 4, insbesondere mittels Kleben oder Ultraschallschweißen, sodass der Angioplastie-Katheter 2 in dem vom Träger 4 und der Verschlussfolie 18 begrenzten Aufnahmeraum keimdicht verpackt ist.

Alternativ oder ergänzend zu den Schritten S3, S4 können Schritt Packen S5 des Trägers in einen Verschlussbeutel und Verschließen S6 des Verschlussbeutels erfolgen, sodass der Träger und der daran gehaltene Katheter keimdicht im Verschlussbeutel verpackt sind.

Es folgt ein Schritt SE Beenden der Herstellung.

### Bezugszeichenliste

- 1: Bereitstellungsanordnung
- 2: Angioplastie-Katheter
- 4; 104; 204; 304; 404: Träger
- 6, 8, 10: Windung
- 12: Hub
- 14: Katheter-Ballon
- 15: Flachseite
- 16; 116; 216; 316; 416: Vertiefung
- 18: Verschlussfolie
- 20: Durchgangsaussparung
- 22: Datenfeld
- 24: Öffnungsabschnitt
- 26: Verbindungsabschnitt
- 28: erster Aufnahmeabschnitt
- 30, 32: Greifmulde
- 34: zweiter Aufnahmeabschnitt
- 36: radial äußerer Randabschnitt
- 37: erste Prägung
- 38: zweite Prägung
- 40: erstes Segment
- 42: zweites Segment
- 44: drittes Segment
- 46: erste Aussparung
- 48: zweite Aussparung
- 50; 450: Hinterschnitt/ Vorsprung
- 52; 252: Rand

- S0: Schritt Herstellung Start
- S1: Schritt Urformen Träger
- S2: Schritt Einlegen Angioplastie-Katheter
- S3: Schritt Überspannen Träger mit Verschlussfolie
- S4: Schritt Verbinden
- S5: Schritt Packen Träger in Verschlussbeutel
- S6: Schritt Verschließen Verschlussbeutel
- SE: Schritt Herstellung Beenden

## Patentansprüche

1. Bereitstellungsanordnung (1; 101; 201; 301; 401) für eine perkutane transluminale Angioplastie, mit einem zu sterilisierenden oder sterilisierten Angioplastie-Katheter (2), der einseitig an einem flächigen Träger (4; 104; 204; 304; 404), in einer oder in mehreren Windungen (6, 8, 10), gehalten ist, und der an einem vorbestimmt proximalen Endabschnitt einen Hub (12) und an einem vorbestimmt distalen Endabschnitt wenigstens einen Ballon (14) aufweist, **dadurch gekennzeichnet, dass** der Träger (4; 104; 204; 304; 404) eine Vertiefung (16; 116; 216; 316; 416) hat, in welcher der Angioplastie-Katheter (2) aufgenommen ist.

2. Bereitstellungsanordnung (1; 101; 201; 301; 401) nach Anspruch 1, **dadurch gekennzeichnet, dass** die Vertiefung (16; 116; 216; 316; 416) einen Randabschnitt (36; 436) hat, an dem die eine oder die mehreren Windungen (6, 8, 10) aufgrund ihrer Biegeelastizität radial außen biegeelastisch abgestützt ist oder sind.

3. Bereitstellungsanordnung (1; 101; 201) nach Anspruch 2, **dadurch gekennzeichnet, dass** die Vertiefung (16; 116; 216) einen mit Bezug zu dem Randabschnitt (36), insbesondere zu der oder den Windungen (6, 8, 10), etwa tangential auslaufenden, ersten Aufnahmeabschnitt (28) oder Halteabschnitt hat, in dem der Hub (12) aufgenommen ist, und der vorzugsweise mit einem Offset zu einer Außenkontur des Hubs (12) ausgebildet ist.

4. Bereitstellungsanordnung (201) nach Anspruch 2 oder 3, **dadurch gekennzeichnet, dass** die Vertiefung (216) einen mit Bezug zu der Windung oder zu einer (10) der Windungen (6, 8, 10) etwa tangential auslaufenden, zweiten Aufnahmeabschnitt (34) oder Halteabschnitt hat, in dem der vorbestimmt distale Endabschnitt oder zumindest der Ballon (14) aufgenommen ist.

5. Bereitstellungsanordnung (201) nach Anspruch 3 oder 4, **dadurch gekennzeichnet, dass** der erste Aufnahmeabschnitt (28) und/ oder der zweite Aufnahmeabschnitt (34) abknickbar ausgebildet ist, sodass der erste Aufnahmeabschnitt (28) in der Weise vom proximalen Endabschnitt oder Hub (12) und/ oder der zweite Aufnahmeabschnitt (34) in der Weise vom distalen Endabschnitt oder Ballon (14) weg geknickt oder weg gefaltet werden kann, dass der proximale Endabschnitt oder Hub (12) und/ oder der distale Endabschnitt oder Ballon (14) frei liegt und einfach gegriffen werden kann.

6. Bereitstellungsanordnung (401) nach einem der Ansprüche 2 bis 5, **dadurch gekennzeichnet, dass** der Randabschnitt (436) maximal einmal umläuft und die Windungen (6, 8, 10) am Randabschnitt (436) als Bündel oder Pulk abgestützt sind.

7. Bereitstellungsanordnung (1; 101; 201; 301) nach einem der Ansprüche 2 bis 6, **dadurch gekennzeichnet, dass** der Randabschnitt (36) mehrfach, insbesondere spiralförmig, umläuft und der Angioplastie-Katheter (2) am Randabschnitt (36) Windung für Windung (6, 8, 10) abgestützt ist.

8. Bereitstellungsanordnung (1; 101; 201; 301; 401) nach einem der Ansprüche 2 bis 7, **dadurch gekennzeichnet, dass** der Randabschnitt (36; 436) zumindest abschnittsweise derart steil und/ oder hinterschnitten (50; 450) ausgebildet ist, dass bei einem Greifen des Angioplastie-Katheters (2) am Hub (12) und einem Abziehen des Angioplastie-Katheters (2) aus der Vertiefung (16; 116; 216; 316; 416) der Angioplastie-Katheter (2) an dem Randabschnitt (36; 436) so geführt ist, dass ein Herausspringen der Windung oder Windungen (6, 8, 10) aus der Vertiefung (16; 116; 216; 316; 416) verhindert ist.

9. Bereitstellungsanordnung (101) nach einem der vorhergehenden Ansprüche, **dadurch gekennzeichnet, dass** an dem Träger (104), insbesondere in einem Bereich des Hubs (12) und/ oder der Windung oder Windungen (6, 8, 10) und/ oder des Ballons, wenigstens eine Greifmulde (30, 32) oder wenigstens eine Durchgangsausnehmung vorgesehen ist, die zumindest abschnittsweise vom aufgenommenen Angioplastie-Katheter (2), insbesondere zumindest abschnittsweise vom Hub (12), von der Windung oder den Windungen (6, 8, 10) oder von dem Katheter-Ballon, überspannt ist.

10. Bereitstellungsanordnung (1; 101; 201; 301; 401) nach einem der vorhergehenden Ansprüche, **dadurch gekennzeichnet, dass** der Träger (4; 104; 204; 304; 404) permeabel für ein Sterilisationsmedium vorgesehen und ausgestaltet ist, vorzugsweise für Wasserdampf und/ oder Ethylenoxidgas, und dass der Träger (4; 104; 204; 304; 404) impermeabel für Keime vorgesehen und ausgestaltet ist.

11. Bereitstellungsanordnung (1; 101; 201; 301; 401) nach einem der vorhergehenden Ansprüche, **dadurch gekennzeichnet, dass** der Träger (4; 104; 204; 304; 404) aus Folie, vorzugsweise Biofolie, tiefgezogen ist oder aus einem Faserwerkstoff, vorzugsweise Biofaserwerkstoff, gegossen ist.

12. Bereitstellungsanordnung (1; 201; 301; 401) nach einem der vorhergehenden Ansprüche, **dadurch gekennzeichnet, dass** die Vertiefung (16; 216; 316; 416) von einer mit dem Träger (4; 204; 304; 404) verbundenen Verschlussfolie (18) überspannt ist, sodass die Vertiefung (16; 216; 316; 416) von der Verschlussfolie (18) keimdicht verschlossen ist, oder dass ein Verpackungsbeutel vorgesehen ist, in dem der Träger angeordnet ist, wobei der Verpackungsbeutel keimdicht verschlossen ist.

13. Bereitstellungsanordnung (1; 201) nach Anspruch 12, **dadurch gekennzeichnet, dass** auf der Verschlussfolie (18) wenigstens ein Kennwert oder eine Kenngröße des Angioplastie-Katheters (2) abgebildet ist, und/oder dass die Verschlussfolie (18) einen Öffnungsabschnitt (24) hat, der mit dem Träger (4) lösbar verbunden ist, und/oder dass die Verschlussfolie einen Verbindungsabschnitt (26) hat, der fester als der Öffnungsabschnitt (24) mit dem Träger (4) verbunden ist und bei gelöstem Öffnungsabschnitt (24) zum Verbleib am Träger (4) vorbestimmt ist.

14. Verfahren zur Herstellung einer Bereitstellungsanordnung (1; 101; 201; 301; 401) für eine perkutane transluminale Angioplastie, insbesondere zur Herstellung einer Bereitstellungsanordnung (1; 101; 201; 301; 401), die gemäß einem der vorhergehenden Ansprüche ausgestaltet ist, **gekennzeichnet durch** Schritte:
- Urformen und/oder Umformen (S1) eines flächigen Trägers (4; 104; 204; 304; 404) mit einer Vertiefung (16; 116; 216; 316; 416);
- Einlegen (S2) eines Angioplastie-Katheters (2), der an einem vorbestimmt proximalen Endabschnitt einen Hub (12) und an einem vorbestimmt distalen Endabschnitt wenigstens einen Katheter-Ballon (14) aufweist, in die Vertiefung (16; 116; 216; 316; 416) derart, dass der Angioplastie-Katheter (2) in einer oder in mehreren Windungen (6, 8, 10) in der Vertiefung (16; 116; 216; 316; 416) aufgenommen und gehalten ist.

15. Verfahren nach Anspruch 14, **dadurch gekennzeichnet, dass** das Urformen und/oder Umformen (S1) durch Tiefziehen einer Folie, vorzugsweise einer Biofolie, oder durch Gießen eines Faserwerkstoffs, vorzugsweise eines Bio-Faserwerkstoffs, erfolgt.

16. Verfahren (1) nach Anspruch 14 oder 15, **gekennzeichnet durch** Schritte:
- Überspannen (S3) der Vertiefung (16; 216; 316; 416) mit einer Verschlussfolie (18); und
- Verbinden (S4) der Verschlussfolie (18) mit dem Träger (4; 204; 304; 404), sodass der Angioplastie-Katheter (2) in einem vom Träger (4; 204; 304; 404) und der Verschlussfolie (18) begrenzten Aufnahmeraum keimdicht verpackt ist; und/oder **gekennzeichnet durch** Schritte:
- Packen (S5) des Trägers in einen Verschlussbeutel; und
- Verschließen (S6) des Verschlussbeutels;
sodass der Träger und der daran gehaltene Angioplastie-Katheter keimdicht im Verschlussbeutel verpackt sind.
